# EUROPÄISCHE PATENTSCHRIFT

(11) **EP 0 993 324 B1**
(45) Veröffentlichungstag und Bekanntmachung des Hinweises auf die Patenterteilung: **08.11.2006**
(21) Anmeldenummer: 99919234.7
(22) Anmeldetag: 14.04.1999
(51) Int. Cl.: A63B 33/00, A61F 9/02

(54) **Adapter zum Befestigen von Brillengläsern**
Adapter for fixing spectacle lenses
Adapteur pour la fixation de verres de lunettes

(30) Priorität: 21.04.1998 DE 29807174 U
(43) Veröffentlichungstag der Anmeldung: 19.04.2000
(73) Patentinhaber: Anton, Wolfgang, Korntal-Münchingen (DE)
(72) Erfinder: Anton, Wolfgang, Korntal-Münchingen (DE)
(74) Vertreter: Winter, Martina
(86) Internationale Anmeldenummer: PCT/EP1999/002512
(87) Internationale Veröffentlichungsnummer: WO 1999/054000

(56) Entgegenhaltungen:
- CA-A- 1 004 403
- DE-U- 29 508 679
- PATENT ABSTRACTS OF JAPAN vol. 009, no. 101 (P-353), 2. Mai 1985 (1985-05-02) & JP 59 224817 A (YOSHIAKI IKEDA), 17. Dezember 1984 (1984-12-17)

## Beschreibung

Die vorliegende Erfindung betrifft einen Adapter zum reversiblen Befestigen eines Brillenglases am Schild einer Schutzbrille nach dem Oberbegriff des Anspruchs 1.

Diese Erfindung bezieht sich auf eine rahmenlose Verbindung von handelsüblichen Schutzbrillen, welche einen durchgehenden Schild oder zwei getrennte, extrem gewölbte Schilde als Augenabdeckung aufweisen, insbesondere Sportbrillen, Sportsonnenbrillen, Taucherbrillen und Arbeitsschutzbrillen, mit Brillengläsern individueller Glasstärke, die vom Träger der Schutzbrille benötigt werden. Für Personen, die eine Brille benötigen, ist es problematisch, eine moderne Schutzbrille zu tragen, welche typischerweise einen Schild in Form einer durchgehenden Scheibe als Augenabdeckung aufweisen. Derartige Schutzbrillen sind bspw. unter den Handelsnamen Oakley-Serie M-Frame, Bolle-Serie Edge und Serie Breakaway, Alpina-Serie Gravity 0 / Bel Air, Swiss Eye etc. erhältlich.

Zwar bieten die meisten Hersteller auswechselbare, in das Brillengestell einsteckbare Rahmenadapter zur Aufnahme von Brillengläsern an. Allerdings haben diese Systeme einige gravierende Nachteile. Bspw. vergrößert sich bei Sportbrillen der Scheitelabstand zwischen Schutzbrille und Auge beträchtlich, in der Regel um etwa 15 mm. Dadurch verringert sich die Abdeckleistung des Schildes bzw. der beiden Schilde, so dass Windwirbel zwischen der Scheibe und dem Auge auftreten können. Außerdem neigen die Schilde bei ungünstiger Witterung zum Beschlagen. Um die Scheiben wieder zu reinigen, muss man die Adapter wiederum entfernen. Außerdem weisen herkömmliche Rahmenadapter ein hohes Gewicht auf.

Bei Taucherbrillen werden seit einiger Zeit Silikat-Brillengläser mittels eines gehärteten Klebers, bspw. eines Epoxid-Klebers mit den Rückflächen der Tauchermaskenscheibe verbunden. Dabei kann sich die chromatische Dispersion verändern. Ferner kann es dadurch zu Problemen kommen, dass bei der Benutzung der Taucherbrille die Tauchermaskenscheibe eine wesentlich niedrigere Temperatur, die von der Temperatur des umgebenden Wassers bestimmt wird, als die Brillengläser aufweist, welche von der Wärme des Gesichts des Brillenträgers erwärmt werden. Diese Temperaturdifferenz bewirkt, dass sich die Brillengläser stärker ausdehnen als die Tauchermaskenscheibe. Die Brillengläser können sich aufgrund dieser Temperaturdifferenz von der Rückfläche der Tauchermaskenscheibe ablösen, da der gehärtete Kleber nicht elastisch ist und die von der unterschiedlichen Wärmeausdehnung herrührende Spannungsbelastung nicht kompensieren kann, sondern reißt.

Klassische Arbeitsschutzbrillen bestehen aus normalen, mit Abdeckteilen ausgerüsteten Brillengestellen und gehärteten Brillengläsern. Die gehärteten Brillengläser sind allerdings sehr teuer und müssen wegen der großen Beanspruchung häufig ausgewechselt werden. Andere Arbeitsschutzbrillen weisen eingesteckte Rahmenadapter auf, wie es oben im Zusammenhang mit den Sportbrillen erläutert wurde. Derartige mit einem Rahmenadapter ausgerüstete Arbeitsschutzbrillen weisen dieselben Nachteile auf wie die oben beschriebenen Sportbrillen.

Ein weiteres großes Problem, welches die Tauglichkeit dieser mit Rahmenadaptern ausgerüsteten Schutzbrillen reduziert, ist der Astigmatismus schiefer Bündel, welcher bei gewölbten Schilden durch die gewinkelte Justierlage der korrigierenden Brillengläser auftritt. Dies zu vermeiden gelingt nur, wenn es möglich ist, die korrigierenden Brillengläser möglichst lotrecht zur optischen Achse zu befestigen, was bei gewölbten Schilden schwierig bis unmöglich ist.

Diese Nachteile schränken die Brauchbarkeit und den Nutzen dieser Schutzbrillen für Brillenträger stark ein.

Das deutsche Gebrauchsmuster DE 295 08 679 U beschreibt einen gattungsgemäßen Adapter zum Befestigen einer Linse an einem Schutzglas, ohne zu offenbaren, wie die Form und der Querschnitt des Adapters beschaffen sind.

Die japanische Patentanmeldung JP 59224817 beschreibt einen Adapter mit einem vergleichsweise aufwendigen und komplizierten Aufbau einer Linsenbefestigung mit einer voluminösen Halterung, einer separaten Wasserdichtung, einem zusätzlichen Abdichtmittel sowie einer separaten Klebeschicht. Die Vielzahl und die Größe der einzelnen Bauteile bedingen, dass sie auf dem Schutzglas sehr aufwendig anzubringen sind und außerdem das Gewicht der fertigen Schutzbrille erheblich erhöhen.

Der vorliegenden Erfindung liegt daher die Aufgabe zugrunde, die Brillengläser einfach, sicher und verträglich, d.h. mit gesteigertem Nutzen für den Brillenträger an den Schilden zu befestigen. Ferner soll ein übermäßiger Gewichtszuwachs vermieden werden.

Die Lösung besteht in einem Adapter mit den Merkmalen des Anspruchs 1. Erfindungsgemäß ist also vorgesehen, dass der Adapter ein Formring aus einem transparenten oder opaken dauerelastischen Material ist, der im Querschnitt keilförmig ausgebildet ist und klebende Flächen zur Verbindung des Brillenglases mit dem Schild aufweist. Der erfindungsgemäße Adapter verbindet die Rückseite des Schildes einer Schutzbrille mit der Vorderseite eines Brillenglases fest und unverrückbar, aber reversibel und damit wieder lösbar. Der erfindungsgemäße Adapter kommt somit gänzlich ohne Aufnahmen oder Rahmen für Brillengläser aus. Das System aus Schutzbrille und Brillenglas ist durch hermetischen Abschluss des Zwischenraumes zwischen Schild und Brillenglas vor dem Beschlagen gesichert. Das Gewicht des erfindungsgemäßen Adapters beträgt nur einen Bruchteil des Gewichts eines herkömmlichen Rahmenadapters. Da der Adapter reversibel klebend ist und somit wieder vom Schild der Schutzbrille und dem Brillenglas abgelöst werden kann, ist er auch wieder verwendbar.

Gegenstand der vorliegenden Erfindung ist ferner ein System bestehend aus einer Schutzbrille mit mindestens einem Schild und daran reversibel befestigten Brillengläsern, wobei die Brillengläser mittels eines erfindungsgemäßen Adapters an dem Schild befestigt sind.

Die Schutzbrille kann eine Sportbrille, eine Sportsonnenbrille, eine Taucherbrille oder eine Arbeitsschutzbrille sein.

Vorteilhafte Weiterbildungen ergeben sich aus den Unteransprüchen. Eine mögliche Konstruktion besteht aus einem Kern aus dem transparenten oder opaken Material, welcher mit klebenden Flächen in Form mindestens einer Schicht aus einem klebenden Material versehen ist. Mit derartigen Adaptern kann man bei einem Plusbrillenglas die starken Durchwölbungen an der Vorderseite kaschieren. Ausschlaggebend für die verwendete Form des Adapters sind immer anatomische bzw. optische Anforderungen.

Der Kern besteht vorzugsweise aus einem Kunststoffmaterial, vorzugsweise Polycarbonat oder Nylon. Dieses Material ist mechanisch belastbar und verträgt auch größere Temperaturunterschiede, ohne zu verspröden.

Im Folgenden werden Ausführungsbeispiele der vorliegenden Erfindung anhand der beigefügten Zeichnungen näher erläutert. Es zeigen:
- Figur 1: eine perspektivische Darstellung der Einzelteile eines Systems aus einem Schild einer Schutzbrille, einer Ausführungsform eines bekannten Adapters und einem Brillenglas;
- Figur 2a: das System aus Figur 1, bei die Einzelteile miteinander verbunden sind;
- Figur 2b: eine optische Durchsicht durch das System aus Figur 2;
- Figuren 3a bis 3c: Seitenansichten verschiedener Ausführungsformen eines Adapters nach dem Stand der Technik;
- Figur 4a: einen Schnitt durch den gewölbten Schild einer Sportbrille mit einem konkaven Brillenglas;
- Figur 4b: einen Schnitt durch den flachen Schild einer Taucherbrille mit einem konvexen Brillenglas;
- Figur 5a: eine perspektivische Ansicht eines im Querschnitt keilförmigen Adapters gemäß der Erfindung;
- Figur 5b: einen Querschnitt durch den Adapters aus Figur 5a.

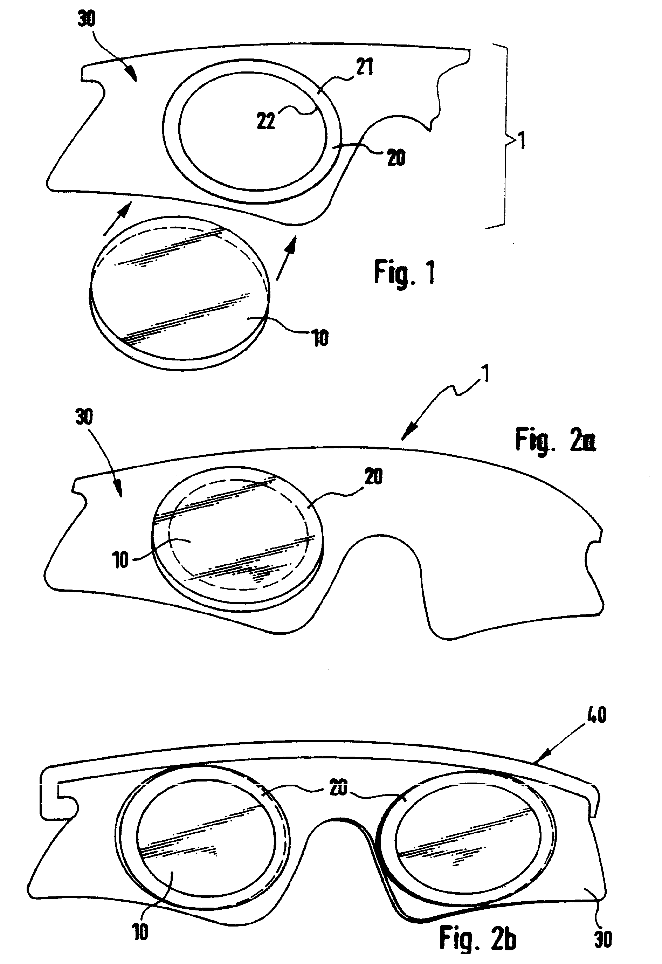

Die Figuren 1 bis 2b zeigen ein System 1 aus einem Brillenglas 10, einen bekannten Adapter 20 in Form eines Formrings und einem Schild 30 einer Schutzbrille 40, bspw. einer Sportbrille, Taucherbrille oder Arbeitsschutzbrille. Das Brillenglas 10 wird frontseitig mit dem Adapter 20 verbunden, dessen Seitenflächen 21, 22 aus einem klebenden Material bestehen. Die Einheit aus Brillenglas 10 und Adapter 20 wird von hinten mit dem Schild 30 verbunden. Die Klebeverbindung ist fest und dauerhaft, aber lösbar.

Hierbei ist es von Vorteil, wenn der Schild 30 von hoher optischer und mechanischer Güte ist und eine möglichst rotationssymmetrische Grundkurve oder Basiskurve aufweist. Die Schilde der meisten verkehrsüblichen Schutzbrillen erfüllen diese Voraussetzungen. Schilde, die lediglich aus einer durchgebogenen Platte bestehen, sind nicht gut geeignet. Die Brillengläser 10 sollten vorzugsweise die gleiche Krümmung oder Basiskurve aufweisen wie der Schild 30. Der Anwender sollte ferner nach spezifizierten Vorgaben wie Augenabstand, Hornhautscheitelabstand, Einschleifhöhe, Glasstärke, insbesondere bei höheren Glasstärken und bei Astigmatismus obliquus, die Briilenglasstärke umrechnen, um den Astigmatismus schiefer Bündel, welcher bei gewölbten Schilden 30 durch die dann nicht mehr lotrechte Stellung der Brillengläser 10 zu erwarten ist, in nicht störenden Grenzen halten. Diese Umrechnung gehört zum Fachwissen eines Augenoptikers und braucht daher nicht näher erläutert zu werden (vgl. Pforte, der Feinoptiker, Teil II).

Es können praktisch alle Arten von Ein- und Mehrstärkenbrillengläsern verarbeiten, wenn auf die jeweiligen augenoptischen Grundforderungen Rücksicht genommen wird.

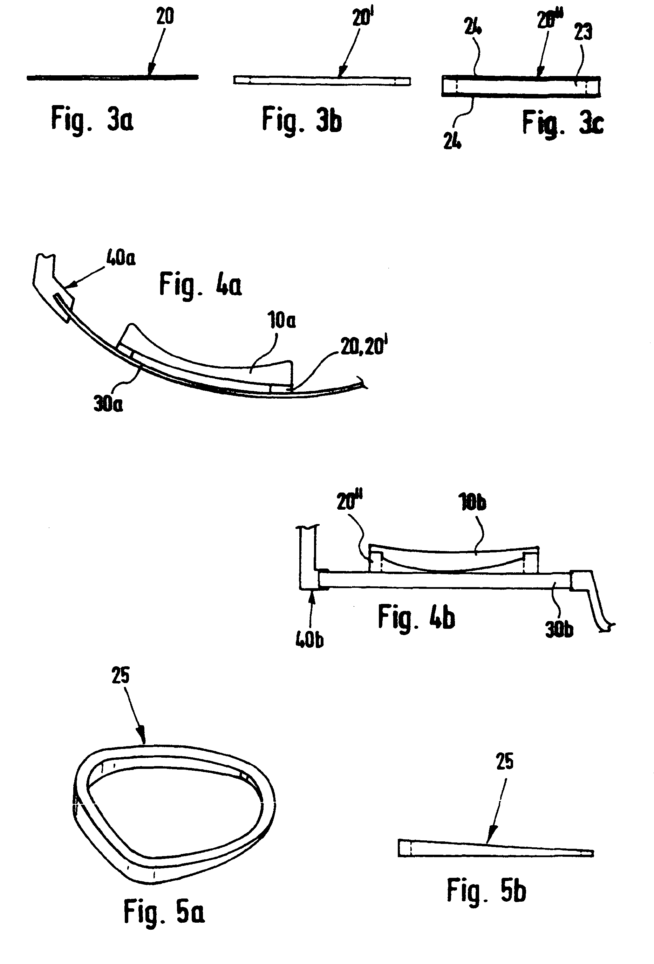

Die Figuren 3a bis 3c zeigen verschiedene Ausführungsformen eines Adapters 20, 20', 20" nach dem Stand der Technik. Die in den Figuren 3a und 3b gezeigten Adapter 20, 20' sind selbstklebende Bänder mit einer Stärke von 0,5 mm bzw. 2 mm aus einem dauerelastischen transparenten oder opaken Material. Geeignet sind bspw. Klebebänder der Fa. 3 M mit den Bezeichnungen VHB 4905 P, 4910 F, 4912 F, 4915 F, 4918 F. Der in Figur 3c gezeigte Adapter 20" weist einen Kern 23 aus einem Kunststoffmaterial wie Nylon oder Polycarbonat auf, der etwa 3,5 mm stark ist und auf beiden Seiten mit einer selbstklebenden etwa 0,5 mm starken Schicht 24 kaschiert ist.

Die Figuren 4a und 4b zeigen Schnitte durch ein konkaves bzw. ein konvexes Brillenglas 10a, 10b, die auf dem gewölbten Schild 30a einer Sportbrille 40a bzw. einem flachen Schild 30b einer Taucherbrille 40b befestigt sind. In Figur 4a ist zu erkennen, daß der gewölbte Schild 30a und die Vorderseite des Brillenglases 10a dieselbe Krümmung bzw. Basiskurve aufweisen, so daß ein flacher Adapter 20, 20' nach dem Stand der Technik verwendet werden kann. Im Gegensatz dazu weist die Vorderseite des konvexen Brillenglases 10b in Figur 4b eine starke Krümmung auf, während der Schild 30b der Taucherbrille 40b flach ist. Dies wird durch Verwendung eines stärkeren Adapters 20" ausgeglichen.

Die Figuren 5a und 5b zeigen einen erfindungsgemäßen Adapter 25, der im Querschnitt keilförmig ist. Der keilförmige Querschnitt erlaubt ebenfalls den Ausgleich unterschiedlicher Krümmungen des Schildes 30 und der Vorderseite des Brillenglases 10.

## Patentansprüche

1. Adapter (20) zum reversiblen Befestigen eines Brillenglases (10) am Schild (30) einer Schutzbrille (40), wobei der Adapter (10) ein im Querschnitt flacher Formring (20) mit einer Dicke zwischen 0,5 bis 2,0 mm ist, der Formring (20) aus einem transparenten oder opaken dauerelastischen Material besteht, und klebende Flächen zur Verbindung des Brillenglases (10) mit dem Schild (30) aufweist, **dadurch** g**ekennzeichnet**, **dass** der Formring im Querschnitt keilförmig ausgebildet ist.

2. Adapter nach Anspruch 1, **dadurch gekennzeichnet, dass** der Formring einen Kern (23) aus dem transparenten oder opaken Material aufweist, welcher mit klebenden Flächen in Form mindestens einer Schicht (24) aus einem klebenden Material versehen ist.

3. Adapter nach Anspruch 1 bis 2, **dadurch gekennzeichnet, dass** der Kern aus einem Kunststoffmaterial, vorzugsweise Polycarbonat oder Nylon besteht.

4. System (1) bestehend aus einer Schutzbrille (40) mit mindestens einem Schild (30) und daran reversibel befestigten Brillengläsern (10), **dadurch gekennzeichnet, dass** die Brillengläser (10) mittels eines Adapters (20) nach einem der Ansprüche 1 bis 3 an dem Schild befestigt sind.

5. System nach Anspruch 4, wobei die Schutzbrille eine Sportbrille, eine Sportsonnenbrille, eine Taucherbrille oder eine Arbeitsschutzbrille ist.

## Claims

1. Adapter (20) for reversibly fixing a spectacle lens (10) to the shield (30) of a pair of safety glasses (40), the adapter (10) being a shaped ring (20) having a flat cross section with thicknesses between 0,5 mm through 2,0 mm which consists of a transparent or opaque, permanently elastic material, said shaped ring having adhesive surfaces for joining the spectacle lens (10) to the shield (30), **characterized in that** said shaped ring (20) has a wedge-shaped cross section.

2. Adapter as defined in claim 1, **characterized in that** the shaped ring (20) has a core (23) of a transparent or opaque material which has adhesive surfaces in form of at least one layer (24) of an adhesive material.

3. Adapter as defined in claim 1 or 2, **characterized in that** the core consists of a plastic material, preferably polycarbonate or nylon.

4. System (1) comprising a pair of safety glasses (40) with at last one shield (30) and spectacle lenses (10) reversibly fixed thereon, **characterized in that** the spectacle lenses (10) are attached to the shield by means of an adapter (20) according to one of the claims 1-3.

5. System as defined in claim 4, in which the safety glasses are a pair of sports glasses, sport sunglasses, diver's goggles or industrial safety glasses.

## Revendications

1. Adaptateur (20) pour la fixation réversible d'un verre de lunette (10) sur le masque (30) de lunettes de protection (40), l'adaptateur (10) étant une bague façonnée (20) de section transversale plate ayant une épaisseur comprise entre 0,5 et 2,0 mm, la bague façonnée (20) étant composée d'un matériau transparent ou opaque à élasticité permanente et présentant des surfaces adhésives pour relier le verre de lunette (10) au masque (30), **caractérisé en ce que** la bague façonnée présente une section transversale conique.

2. Adaptateur selon la revendication 1, **caractérisé en ce que** la bague façonnée présente une âme (23) en matériau transparent ou opaque qui est muni de surfaces adhésives sous la forme d'au moins une couche (24) d'un matériau adhésif.

3. Adaptateur selon les revendications 1 à 2, **caractérisé en ce que** l'âme se compose d'une matière plastique, de préférence du polycarbonate ou du nylon.

4. Système (1) composé de lunettes de protection (40) comprenant au moins un masque (30) et des verres de lunette (10) fixés à celui-ci de manière réversible, **caractérisé en ce que** les verres de lunette (10) sont fixés au masque au moyen d'un adaptateur (20) selon l'une des revendications 1 à 3.

5. Système selon la revendication 4, les lunettes de protection étant des lunettes de sport, des lunettes de soleil à usage sportif, des lunettes de plongée ou des lunettes de protection de travail.
